Europäisches Patentamt

European Patent Office

Office européen des brevets

(19)

(11) Publication number: **0 379 850**
**A1**

# EUROPEAN PATENT APPLICATION

(21) Application number: **89850431.1**

(22) Date of filing: **11.12.89**

(51) Int. Cl.⁵: **B31D 1/02, A61F 13/15, C09J 7/04**

(30) Priority: **30.12.88 SE 8804713**

(43) Date of publication of application:
**01.08.90 Bulletin 90/31**

(84) Designated Contracting States:
**GR**

(71) Applicant: **Mölnlycke AB**

**S-405 03 Göteborg(SE)**

(72) Inventor: **Aronsson, Anders**
**Tunnlandsgatan 18**
**S-421 38 Västra Frölunda(SE)**
Inventor: **Johansson, Dan**
**Hallstenshagen 27**
**S-421 38 Västra Frölunda(SE)**
Inventor: **Olsson, Lennart**
**Aprilgatan 72**
**S-415 15 Göteborg(SE)**

(74) Representative: **Kierkegaard, Lars-Olov et al**
**H. ALBIHNS PATENTBYRA AB P.O. Box 3137**
**S-103 62 Stockholm(SE)**

(54) **A method for manufacturing fastening tape and fastening tape manufactured in accordance with the method.**

(57) The present invention relates to a method for manufacturing adhesive tapes (1, 2) from an elongated material web (3) which is coated with an adhesive (6, 8) at least along the side margins thereof, and which has a non-adhesive, straight band (7) extending centrally in the direction of the longitudinal axis of the web. In accordance with the invention the web (3) is divided longitudinally by means of two wave-shaped line-cuts (10,11; 10′,11′) which are positioned so that the line-cuts will not intersect one another within the region of the non-adhesive band (7), such that the wave crests of a first line-cut (11; 11′) and the troughs of a second line-cut (10; 10′) lie within the confines of the non-adhesive band, whereas the wave troughs of the first line-cut (11; 11′) and the wave crests of the second line-cut (10; 10′) lie externally of the non-adhesive region. The web is also divided transversely by means of line-cuts (12; 12′) which extend transversely to the long axis of the web, from the centre of the crests and troughs of the two longitudinally extending line-cuts (10,11; 10′, 11′), which lie externally of the non-adhesive band, to the side edges of the elongated web.

The invention also relates to a fastening tape (1,2) manufactured in accordance with the method.

FIG. 1

# A method for manufacturing fastening tape and fastening tape manufactured in accordance with the method

The present invention relates to a method for the manufacture of fastening tape from an elongated web of material, which is coated with adhesive on one side thereof, at least along the edge-margins of the web and along a central, longitudinally extending web-part, and in which a non-adhesive, straight band or strip is provided in the central part, in the longitudinal direction of the web.

The invention also relates to fastening tape manufactured by the inventive method.

The inventive fastening tape is primarily intended for fastening together parts of a so-called all-in-one diaper such as to form a pants-like configuration. Such diapers include a front part and a back part, the side-portions of which overlap one another in the waist-region of the user, and are fastened to one another with the aid of fastening tapes. In the case of the type of fastening tapes to which the invention relates, one end of the tapes is adhesively bonded to the front or back part of the diaper, at a location adjacent one side margin thereof, while the other end of the tape is either coated with a release agent or is left free of adhesive, so as to form a non-adhesive finger grip. The length of tape extending between the tape-ends is coated with an adhesive of such adhesive qualities as to provide a refastenable tape.

At present, fastening tapes of this kind are manufactured by coating an elongated web of material with an adhesive substance, with the exception of a central, longitudinally extending band or strip, and by dividing the thus coated web longitudinally, in the centre of the band or release agent and then transversely across the web, such as to form pairs of rectangular fastening tapes, which are subsequently fixed in pairs onto the respective diapers. It is also usual to fold the side-portions the web into a Z-configuration, and to apply release agent in the Z-folds, so as to facilitate unfolding of the fastening tapes produced from the web.

Thus, this method makes possible the manufacture of fastening tapes which can be fastened directly, in pairs, onto the diapers in the process of diaper-manufacture in the diaper-production line, in a relatively simple manner.

It has been found, however, that subsequent to putting-on a diaper on which fastening tapes are applied, the non-adhesive end of such rectangular fastening tapes is liable to extend outwardly from the remainder of the fastening tape. A few cases have been documented in which diaper-wearing children have cut themselves on an outwardly projecting corner of the grip end of such as fastening tape.

The invention is intended to avoid the aforesaid drawback by means of a method which will enable fastening tapes to be formed in the absence of such outwardly projecting corners, which fastening tapes can be fastened to the side-portions of diapers without creating stoppages or other disturbances in the diaper-production line. The invention also relates to a fastening tape manufactured in accordance with the inventive method.

This object is achieved in accordance with the invention by means of a method characterized by the features set forth in Claim 1, and by a fastening tape characterized by the features set forth in Claim 4. Thus, because the non-adhesive end-parts are located solely on the crests and troughs of the line-cuts produced in the grip-ends of respective fastening tapes, no end-part which projects from the tape will present sharp corners which are liable to cut or chafe the skin of a diaper-wearing child. The inventive method is well suited for use in a production line for the production of diapers of the kind to which application of the invention relates, i.e. all-in-one diapers, whose respective side-portions include a fastening tape for releasably fastening together the waist-parts of the side-portions, without needing to modify excessively the equipment or machinery used at present to produce rectangular fastening tapes and reapply these tapes or tabs to diapers in existing diaper-production lines.

These and other features of the invention, together with advantages afforded thereby, will be apparent from the following detailed description of embodiments of the method for manufacturing a fastening tape in accordance with the invention, this description being made with reference to the accompanying drawings, in which:

Fig. 1 illustrates schematically from above a linear array of fastening tapes manufactured in accordance with one embodiment of the inventive method;

Fig. 2 is a cross-sectional view taken on the line II-II in Fig. 1;

Figs. 3-5 illustrate various wave-shapes which can be imparted to the grip-ends of the fastening tapes in Fig. 1;

Fig. 6 is a view, similar to Fig. 1, of a linear array of fastening tapes manufactured by means of a second embodiment of the inventive method, in which the longitudinally extending, undulatations or wave-forms are displaced in phase relative to one another;

Figs. 7-9 illustrate different wave-shapes which can be imparted to the grip-ends of the fastening tapes in Fig. 6;

Fig. 10 illustrates schematically the construction of cutting apparatus by means of which the wave-shaped line-cuts can be made; and

Fig. 11 illustrates schematically a machine for the manufacture of all-in-one diapers in which the fastening tapes are manufactured by means of the inventive method.

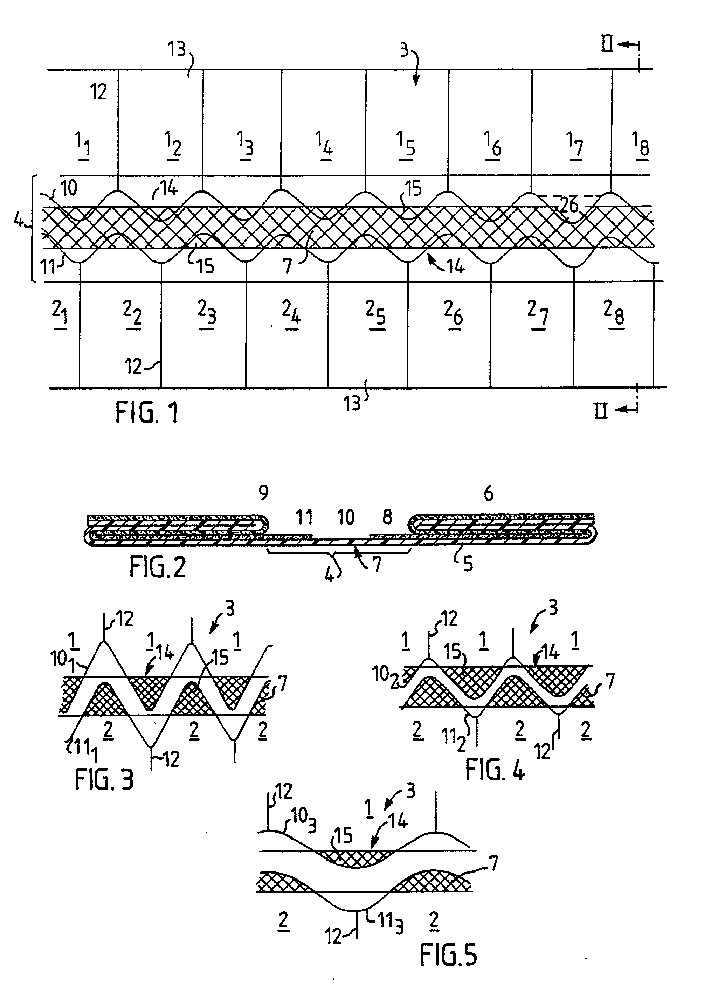

Figs. 1 and 2 illustrate schematically and from above a linear array of fastening tapes 1, 2 manufactured by means of the inventive method from a base web 3 of material, which is coated with an adhesive and a release agent. As will be best seen from Fig. 2, the outer parts of the web 3 are folded into a Z-like configuration, while leaving a central part 4 of the web unfolded. The web 3 may consist of a plastic or paper strip 5 and has longitudinally along its centre part a non-adhesive band 7. The the side margins of the web, or strip, are coated with an adhesive 6 of the kind which will ensure that the said side margins will adhere reliably to a suitable substrate. Except for the central non-adhesive band 7, the central part of the web 3 or strip 5 is coated with an adhesive 8 of the kind which is capable of connecting said web part releasably to a suitable substrate. The adhesive substances 6, 8 are preferably a glue of the PSHM-type, i.e. a pressure sensitive hot-melt glue. The strip 5 is preferably coated with a release agent 9, for instance silicone, between the adhesive coatings.

The array or row of fastening tapes $1_1$-$1_8$, $2_1$-$2_8$ are produced from the aforesaid web 3, by dividing the web longitudinally by means of undulating or wave-shaped line-cuts 10, 11 and a plurality of transverse line-cuts 12. The longitudinally extending line-cuts 10, 11 have a sinusoidal wave shape with waves of mutually the same amplitude and length. The line-cuts are also placed within the region of the non-adhesive band 7, although without intersecting each other. For the sake of illustration, the non-adhesive band 7 is hatched in the drawings, and it will be seen that the wave crests of the line-cut 10 and the wave troughs of the line-cut 11 lie outside the confines of band 7. The transverse line-cuts 12 are transverse to the longitudinal axis of the web and extend from respective wave crests of the line-cut 10 and respective wave troughs of the line-cut 11 to respective side-edges of the web 3.

By dividing the web 3 and the fastening tapes 1,2 in the aforedescribed manner there are produced resealable fastening tapes $1_1$-$1_8$, $2_1$-$2_8$, which include an anchoring-end 13 and a grip-end 14, the outermost part of the grip-end comprising a non-adhesive part 15. It will be observed that the corners of each grip end 14, these corners being defined by each line-cut 12 transversely to the longitudinal line-cut 10 or 11, lies within an adhesive-coated part of the web 3, and consequently that part of the grip-end 14 which is liable to project outwardly when the fastener tab is used and which is formed from the non-adhesive end-part of the adhesive strip 1, 2, will not include sharp corners. When an adhesive tape produced in accordance with the present invention is used to fasten together the side-portions of an all-in-one diaper, there is consequently no risk of an outwardly protruding part of the grip-end 14 of chafing or cutting the skin of a child wearing a diaper.

As will be understood, instead of providing an adhesive-free band 7, the web 3 may alternatively be coated with a band of release agent, either directly on the web or on top of an adhesive layer. The expression "non-adhesive band" as used in the present document is intended to cover both of these variants.

Figs. 3-5 illustrate only a small and central part of a web 3 according to Fig. 1, in which longitudinally extending undulating cuts of mutually different wave-shapes have been made, and in which those parts of the non-adhesive band 7 incorporated in the adhesive tapes 1, 2 cut from the band have been shown in hatch, for the sake of illustration.

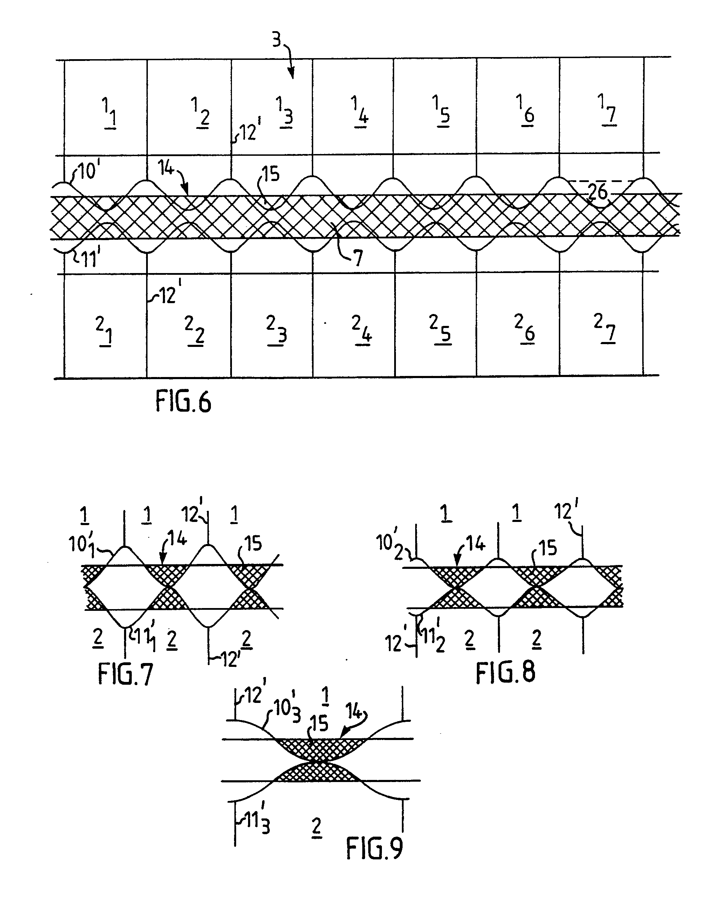

The wave-shaped line-cuts $10_1$ and $11_1$ in Fig. 3 are located symmetrically around the upper and the lower demarcation line of the band 7, similar to the line-cuts 10 and 11 in Fig. 1, and the wave-shapes cut from the web have mutually the same wave-length. The waves of the Fig. 3 embodiment, however, have a greater amplitude than the waves of the Fig. 1 embodiment. In the embodiment illustrated in Fig. 4, the line-cuts $10_2$ and $10_3$ are no longer symmetrical around the upper and lower demarcation lines of the band 7, but are displaced towards the centre of said band. In this case, a larger part of the wave-shaped line-cuts will lie within the band 7 and the grip-ends of the adhesive tapes 1, 2 will include an outer non-adhesive area which is greater than that obtained when the waves are positioned in the manner illustrated in Fig. 1. It will be understood, however, that if desired, the waves can also be displaced towards the edges, so as to reduce the size of the non-adhesive outer-part of the grip-ends. The wave-length of the waves of the Fig. 5 embodiment is twice the wave-length of the waves of the embodiments illustrated in Figs. 1, 3 and 4.

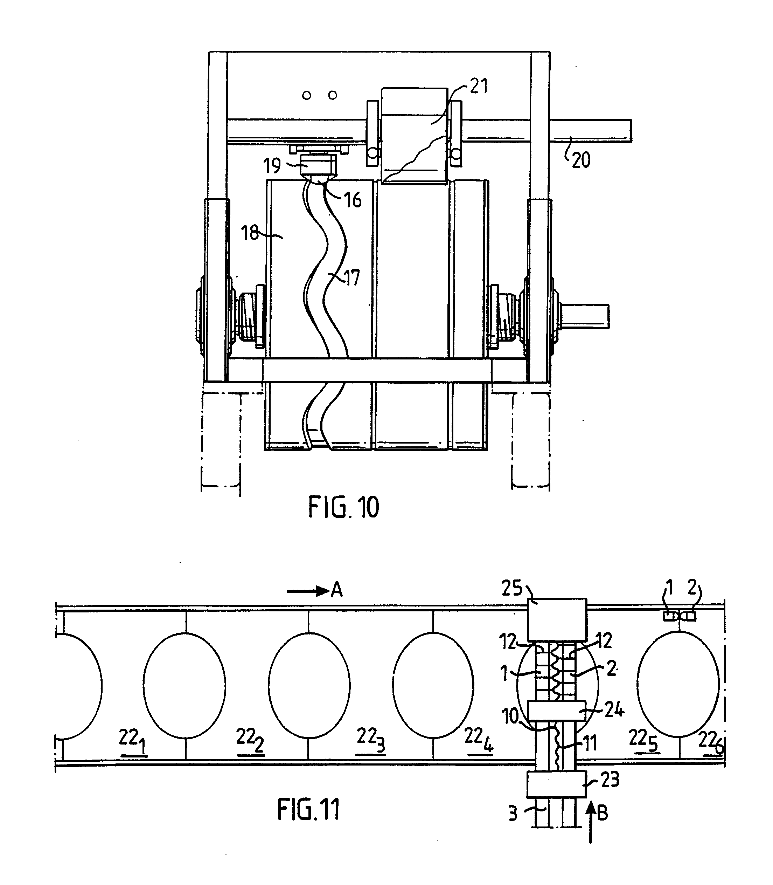

The illustrations of Figs. 3 to 5 are intended solely to illustrate certain possibilities of varying the wave-shapes of adhesive tapes manufactured in accordance with the aforedescribed method. The desired width of the adhesive tapes, however, will be determined by factors other than desired wave-shapes, and consequently the wave-length will constitute a non-variable construction parameter for variation of the wave-shape. Thus, in practice, the wave-amplitude and the positioning of the symmetry lines of the waves in relation to the non-adhesive band will be variable, so as to obtain on the grip-ends of respective adhesive tapes a non-adhesive area of desired dimensions. As will be understood from the following description with reference to Fig. 11, other considerations of a manufacturing/technical nature also influence the possibilities of varying these factors. It will also be understood that the width of the non-adhesive band can be increased when the aforesaid possibilities of varying the wave-shapes are insuffi cient in themselves to provide a non-adhesive area of desired extension.

In this respect, it is pointed out that those parts of the line-cuts 10, 11 which lie outside the area of the non-adhesive band need not be curved, and that the important factor in this regard is that those parts of the line-cuts 10,11 which lie inwardly of the band 7 are curved. The invention thus also embraces composite wave shapes in which those parts of the line-cuts 10,11 which lie externally of the non-adhesive band include straight lines. Waves of regular wave-shape are preferred, however, for manufacturing reasons.

The adhesive tapes 1, 2 illustrated in Figs. 6-9 are manufactured in accordance with a second embodiment of the inventive method. The only difference between this second embodiment and the embodiment illustrated in Figs. 1-5 is that the waves formed by the line-cuts $10'$, $11'$ are mutually displaced in phase through one half of a wavelength. Consequently, the wave crests on the line-cut $10'$ will lie opposite the wave troughs on the line-cut $11'$, and the wave crests on the line-cut $11'$ will lie opposite the wave crests on the line-cut $10'$. This means, in turn, that the transverse line-cut $12'$, which together with dividing line $11'$ defines the adhesive tabs $2_1$-$2_7$ in Fig. 6, lie opposite the transverse line-cuts $12'$, which together with the line-cut $10'$ define the adhesive tabs $1_1$-$1_7$ in Fig. 6. The web 3 and the adhesive tabs $1_1$-$1_7$, $2_1$-$2_7$ are identical with corresponding elements in Fig. 1 and have therefore been identified with the same reference signs.

Figs. 7-9 illustrate variations in the wave-shapes of the line-cuts $10'$ and $11'$, these variations corresponding in principle to the variations of the line-cuts 10 and 11 illustrated in Figs. 3-5. The wave-variations possibilities in the case of this embodiment, however, are limited by the fact that the waves may not intersect one another. Consequently, the waves may not extend beyond the centre of the non-adhesive band.

It will be understood that the possibilities of varying the wave-shapes are increased when it is not necessary for the wave-shapes of the two longitudinally extending line-cuts to be identical with one another. However, when practising the present invention it is essential that the wave-shapes presented by said line-cuts are mutually identical, and consequently those possibilities which are available when this requirement does not exist will not be discussed in detail.

Fig. 10 illustrates an embodiment of a cutting apparatus particularly suited for obtaining the longitudinally extending line-cuts 10 and 11 illustrated in Figs. 1, 3-5. This apparatus comprises a ball 16 which is urged against the edges of a groove 17 extending peripherally around a rotatable roller 18. When a web 3 is fed into the nip defined between the roller 18 and the ball 16, a slot having the shape of the groove 17 will be cut in the web. The ball 16 is journalled on a holder 19, which in turn is carried by a resilient arm (not shown) which is biased at a given force in a direction towards the groove 17, by suitable means herefor. The arm is attached appropriately to a frame or the like, in a manner such that the arm will move substantially parallel with the roller axis. Movement of the ball 16 in this direction is thus guided by the groove 17. The apparatus includes a feed roller 21 which is carried by a shaft 20 operative to feed the web into the nip between the ball and the groove. For a more detailed description of the principle construction of such a "ball knife", reference is made to Swedish Patent Specification 7805988-8.

Fig. 11 illustrates schematically the application of the inventive method in a production line for the manufacture of all-in-one-diapers. These diapers $22_1$-$22_6$ are conveyed side-by-side on a conveyer belt which moves in the direction of arrow A. A material web 3, similar to the material web shown in Fig. 1, is conveyed over the rows of diapers 22 at right angles to the diaper feed direction, in some suitable manner, as indicated by the arrow B. The web 3 is conveyed so that the adhesive-coated side and release-agent coated side thereof faces towards the diapers, and therewith can be supported, for instance, by a vacuum-type conveyer belt not shown. The web thereafter passes through cutting apparatus 23, preferably of the ball-knife-type, which produces the aforesaid two longitudinally extending line-cuts 10 and 11. The web 3 is then passed by means of a suitable conveyer to a further cutting apparatus 24, in which the transversal cuts 12 are made. The thus produced adhesive tapes 1, 2 are finally introduced in pairs into an arrangement 25 which includes means for gripping each adhesive tape 1, 2 and bringing said tapes into alignment with one another and with means for subsequently pressing the tapes 1, 2 against underlying diapers, such as to fasten pairs of tapes onto respective diapers. One such pair of adhesive tapes fastened to a diaper is shown to the right in Fig. 11.

It will be seen that the equipment required to practise the present invention can be readily included in an existing diaper production line, solely by component substitution and therewith without changing the principle construction of the production line.

When the amplitude and positioning of the longitudinally extending line-cuts 10, 11 are such that the waves formed do not overlap one another in the transverse direction, it will be seen that the wave crests of one line-cut can be caused to lie opposite the wave troughs of the other line-cut, before introducing the web 3 into the cutting apparatus 24. This displacement of the waves can be readily achieved by causing one half of the web divided by the cuts 10, 11 to move through a distance which is longer by half a wave-length than the distance moved by the other half of the web, between the cutting apparatus 23 and 24. This movement of the web parts can be achieved simply, by passing said web-part over a roller or like device. In this case, the cutting apparatus 24 need only make a cut transversely across the whole width of the web 3, instead of two longitudinally displaced cuts, and the arrangement 25 need not include means for bringing the adhesive tapes 1, 2 into line with one another. In view of the simplification in the design of the apparatus-components afforded by such a wave-shape, it is preferred that the longitudinally extending or cuts 10, 11 are configured so that the resultant waves will not overlap one another in the transverse direction.

When the cutting apparatus 23 is constructed to produce the longitudinally extending line-cuts 10' and 11' according to Fig. 6 instead of the longitudinally extending line-cuts 10 and 11 according to Fig. 1, the transverse line-cuts made by the cutting apparatus 24 will lie opposite one another, and hence the components 24, 25 of one such cutting apparatus 23 may be identical with corresponding components now used to produce adhesive tapes with straight grip-ends. A ball-knife cutter, however, is less suitable for producing such longitudinally extending line-cuts 10', 11' and consequently when practising this embodiment of the inventive method it is preferred to use cross-knives which act against a smooth roller. When practising this embodiment, the longitudinally extending line-cuts need not be made prior to the transverse line-cuts, since it is possible, for instance, to cut the web longitudinally immediately prior to fixing the adhesive tapes to respective diapers.

It will be understood that although the inventive method has been described with reference to its application with all-in-one-diapers, the method can also be applied advantageously in all cases where it is desired to produce pairs of adhesive tapes which include non-adhesive or release-agent coated, rounded grip-ends.

As will be seen from Figs. 1, 3-5 and 6-9, the adhesive tapes 1, 2 produced in accordance with the inventive method have a grip-end 14 which is terminated in a rounded part 26, the outer portion of which is free of adhesive and which has been shown by broken lines in Figs. 1 and 6 in respect of the adhesive tape 1₇. Furthermore, the demarcation line between the non-adhesive band 7 and the adhesive coating 8 (see Fig. 2) is a straight line which extends perpendicularly to the late ral defining lines 12 or 12' of a respective adhesive tape. The band 7 extends inwardly, i.e. in a direction towards the opposite end of the grip-end of said tape, through a distance which is smaller than the extension of the rounded part 26 in this direction. This will ensure that any sharp corners on the adhesive tape 1, 2 will lie within that part of the tape which is coated with adhesive, thereby avoiding the presence of out wardly projecting sharp corners when using the adhesive tape.

It will be understood that the described embodiments of the method and the described inventive adhesive tape can be modified within the scope of the invention. For instance, the longitudinally extending cuts may have wave-shapes different to those described. Furthermore, in the case of applications different to those described above, the cuts need not necessarily be made in webs that have been folded. Consequently, the invention is only limited by the scope of the following claims.

## Claims

1. A method for manufacturing adhesive tapes (1, 2) from an elongated material web (3) which is coated on one side thereof with an adhesive (6, 8) at least along the side margins said web, and which has a non-adhesive, straight band (7) extending centrally in the direction of the longitudinal axis of the web, characterized by dividing the web (3) longitudinally by means of two wave-shaped line-cuts (10, 11; 10',11'), the waves of which have mutually the same wave-length; positioning the line-cuts within the region of the non-adhesive band (7) such that said line-cuts will not intersect one another and such that the wave crests of a first line-cut (11; 11') and the wave troughs of a second line-cut (10; 10') will lie within the region of the non-adhesive band, said wave-parts having a rounded shape at least within said non-adhesive region, whereas the wave troughs of the first line-cut (11; 11') and the wave crests of the second line-cut (10; 10') lie externally of the non-adhesive region; and by dividing the web transversely by means of line cuts (12; 12') which extend transversely from the centre of the wave crests and the wave troughs of the two longitudinally extending line-cuts (10, 11; 10′, 11′) lying externally of the non-adhesive band to the side-edges of the elongated web.

2. A method according to Claim 1, characterized by configuring the two longitudinally extending line-cuts (10, 11) so that the crests and the troughs of the resultant waves will lie opposite one another in the transverse direction of the web.

3. A method according to Claim 1, characterized by configuring the two longitudinally extending, wave-shaped line-cuts (10′, 11′) so that the waves will be displaced through one half of a wave length relative to one another and so that the wave crests of one line-cut will lie opposite the wave troughs of the other line-cut.

4. An adhesive tape produced by means of a method according to any one of Claims 1 to 3 from an elongated material web (3) which is coated with an adhesive (6, 8) along at least the edge margins of said web and which has a non-adhesive, straight band (7) extending centrally in the longitudinal direction of said web, and which adhesive tapes (1, 2) include an anchoring end (13) and a grip end (14), characterized in that the grip-end (14) of the tape has a rounded outer portion (26) which is located centrally in relation to the side-edges (12, 12′) of the tape, and which terminates in a non-adhesive area; and in that the corners of the grip-end lie in an adhesive region of the adhesive tape.

FIG. 1

FIG. 2

FIG. 3

FIG. 4

FIG. 5

EP 0 379 850 A1

FIG.6

FIG.7

FIG.8

FIG.9

FIG. 10

FIG.11

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | EP,A1, 0 292 970 (UNI-CHARM CORPORATION) 30 November 1988, *See fig. 3 detail 22 and 23 | 4 | B 31 D 1/02 A 41 B 13/02 C 09 J 7/04 |
| X | EP,A2, 0 233 704 (THE PROCTER & GAMBLE COMPANY) 26 August 1987 *See fig. 3 detail 62, fig 4 detail 4 | 4 | |
| A | FI,B, 58 752 (AMERPLAST SUOMINEN & CO) 31 December 1980 | 1-3 | |
| A | FR,A, 2 485 893 (VEREINIGTE PAPIERWERKE SCHICKEDANZ & CO) 19 May 1981 *See the whole document* | 4 | |
| A | US,A, 4 037 602 (HAWTHORNE) 26 July 1977 | 4 | TECHNICAL FIELDS SEARCHED (Int Cl 4) A 41 B B 31 D C 09 J G 09 F |

The present search report has been drawn up for all claims

| Place of search | Date of completion of the search | Examiner |
|---|---|---|
| STOCKHOLM | 22.03.1990 | Björn Lindkvist |